Europäisches Patentamt

European Patent Office

Office européen des brevets

(19)

(11) Publication number: **0 105 677**
**B1**

(12) EUROPEAN PATENT SPECIFICATION

(45) Date of publication of patent specification: **10.12.86**

(21) Application number: **83305653.4**

(22) Date of filing: **22.09.83**

(51) Int. Cl.⁴: **A 61 N 5/04,** A 61 N 1/06, A 61 N 1/32, H 01 Q 13/08

(54) Endotract antenna device for hyperthermia.

(30) Priority: 27.09.82 JP 167975/82
27.09.82 JP 167976/82
27.09.82 JP 167978/82

(43) Date of publication of application:
**18.04.84 Bulletin 84/16**

(45) Publication of the grant of the patent:
**10.12.86 Bulletin 86/50**

(84) Designated Contracting States:
**DE FR GB IT**

(56) References cited:
EP-A-0 012 122
EP-A-0 048 402
WO-A-81/03616
DE-A-2 921 856
DE-A-3 011 322
GB-A-1 238 200
US-A-4 204 549
US-A-4 290 435

SOVIET INVENTIONS ILLUSTRATED, Section
P.Q. week D 36, October 14, 1981 DERWENT
PUBLICATIONS LTD:, London, p.34

(73) Proprietor: KUREHA KAGAKU KOGYO
KABUSHIKI KAISHA
9-11 Horidome-cho 1-chome Nihonbashi Chuo-
ku.
Tokyo (JP)

(72) Inventor: Sogawa, Akira
Dai-Ni Kureha-Soh 3-10-12 Nerima
Nerima-ku Tokyo (JP)
Inventor: Inokuchi, Kiyoshi
1-3-47 Terazuka Minami-ku
Fukuoka-shi Fukuoka-ken (JP)
Inventor: Sugimachi, Keizo
3-19-10 Hatta Higashi-ku
Fukuoka-shi Fukuoka-ken (JP)
Inventor: Kai, Hidenobu
6-14-5 Befu Jonan-ku
Fukuoka-shi Fukuoka-ken (JP)
Inventor: Hotta, Tetsuya
5-15-6 Honcho
Hoya-shi Tokyo (JP)
Inventor: Kawai, Yoshio
3-12-10 Kichijoji-Higashicho
Musashino-shi Tokyo (JP)

(74) Representative: Haigh, Charles Roy et al
J.A. KEMP & CO. 14 South Square Gray's Inn
London WC1R 5EU (GB)

Courier Press, Leamington Spa, England.

## Description

This invention concerns an endotract antenna device for hyperthermia and, more specifically, it relates to an endotract antenna device having a microwave radiation antenna to be used in the hyperthermia therapy of tumors or the like on the organs inside the body.

In hyperthermia therapy for carcinoma, which utilizes the property of the cancer cells that they are less resistant than normal cells against heat or elevated temperature, a microwave radiation antenna is used to warm the lesion for the therapy. Such an antenna device is shown in GB—A—2 045 620 to have an antenna in a surrounding polymer element. It is said that liquid cooling may be applied to the metal shaft of the device.

It is desired that the radiation antenna is as thin as possible so that it may be inserted deeply into the endotract of the body for the therapy of organs inside the body, for example, a digestive organ, and a sort of linear dipole antenna has been employed so far for such a purpose.

However, since the conventional dipole antenna is designed with consideration of the dimensional stability, and lacks flexibility, it is difficult to dispose the conventional linear dipole antenna in direct contact with a surface of the organ at the lesion, and there is fear that gases or fluid may remain in the gap between the antenna and the surface of the organ.

As a result, the electromagnetic energy emitted from the microwave antenna of such an endotract antenna device is absorbed by the body fluid in the gap, or reflected at the gap, and cannot always serve for effective and uniform warming of the lesion and for effective hyperthermia therapy of the lesion.

Further, since the conventional dipole antenna comprises a pair of electrodes different from each other in shape, it is difficult to predetermine the field pattern of electromagnetic energy emitted therefrom and, accordingly, to adequately situate the antenna relative to the lesion area.

According to the present invention, there is provided an endotract antenna device for hyperthermia comprising an antenna for radiating microwaves, and an envelope made of a polymer material surrounding said antenna, characterised in that the envelope is a balloon-like member made of a flexible and elastic polymeric thin film and forming a chamber of variable volume, and by means for feeding and draining a cooling liquid to and from the inside of said balloon-like member.

Preferably, the microwave radiating antenna comprises two elongated and identically-shaped conductors arranged on a straight line and slightly spaced apart from each other, each having a length equal to an integral multiple of about 1/4 of a wavelength of the microwaves to be emitted from said antenna.

In preferred embodiments, the microwave radiation antenna comprises a pair of flexible tubular members made of electrically conductive material for microwave radiation mounted to the outer circumference of a rod-like member made of a flexible and insulating material.

The invention allows provision of an endotract antenna device capable of closely contacting and effectively warming the lesion at the wall of the tract or lumen while avoiding localized overheating. The microwave antenna can be situated surely at a desired position in the tract or lumen, and is highly flexible and effectively applicable to the hyperthermia therapy for the lesion in the tract or lumen.

In order that the invention may be more clearly understood, the following description is given, by way of example only, with reference to the accompanying drawings, in which:

Figure 1 is an explanatory view of an endotract antenna device for hyperthermia as a preferred embodiment of this invention;

Figure 2 is an explanatory sectional view of a preferred embodiment of a microwave antenna contained in the endotract antenna device for hyperthermia;

Figure 3 is an explanatory view of the radiation pattern of the antenna shown in Figure 2; and

Figures 4 and 5 are explanatory views respectively for modified preferred embodiments of the microwave antenna contained in the endotract antenna device for hyperthermia.

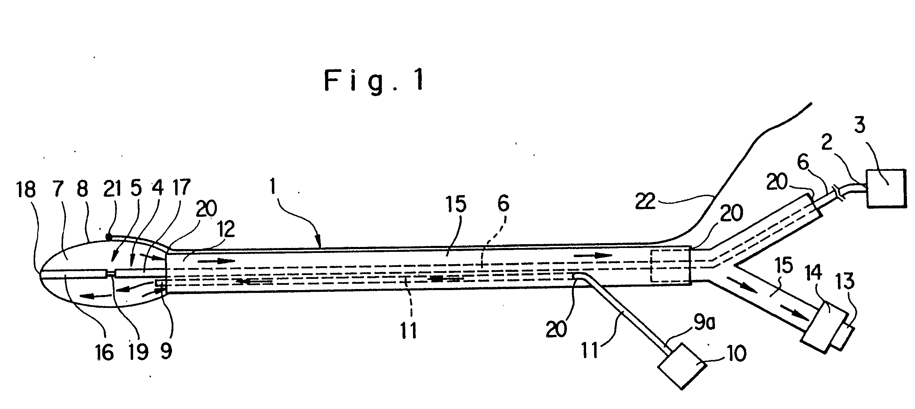

Referring to Fig. 1, an endotract antenna device for hyperthermia 1 comprises a coaxial cable 6 for the microwave transmission connected at one end 2 to a microwave oscillator or generator 3 which can continuously generates microwaves at a frequency between 300—3,000 MHz, for example, 915 MHz and formed at the other end 4 with a sort of linear dipole antenna 5, a balloon 8 made of a flexible and elastic polymeric thin film and forming a chamber 7 of a variable volume which surrounds the microwave radiation antenna 5 and receives purified water for cooling, a feed tube 11 opened at one end 9 thereof to the water-containing chamber 7 and communicated at the other end 9a thereof with a feed pump 10 so as to feed the purified water into the chamber 7, and a draining tube 15 connected at one end 12 thereof to the balloon 8 and opened at the other end 13 thereof by way of a throttling device 14 for the water pressure control so as to drain the water from the balloon 8. In the drawing, the pump 10, the feed tube 11, the throttling device 14 and the draining tube 15 constitute means for feeding and draining the cooling water.

The frequency of the microwaves generated from the oscillator 3 to be applied to tumors at the wall of the tract is usually in the order of between 300—3000 MHz (wave length ($\lambda$) is in the order of 10—1 cm in the body fluid or at the lesion). The frequency of the microwaves may be selected from a plurality of oscillation frequencies that can be generated from the oscillator 3 depending on the size of the antenna 5. The output power of the oscillator 3 may be in the order of 10 to 200 watt for example.

The antenna 5 comprises tubular conductors 16 and 17 each of the identical shape and separated from each other by an insulator 19. Instead of providing the tubular conductor 16, the top end of the central conductor may be exposed about by the length of λ/4.

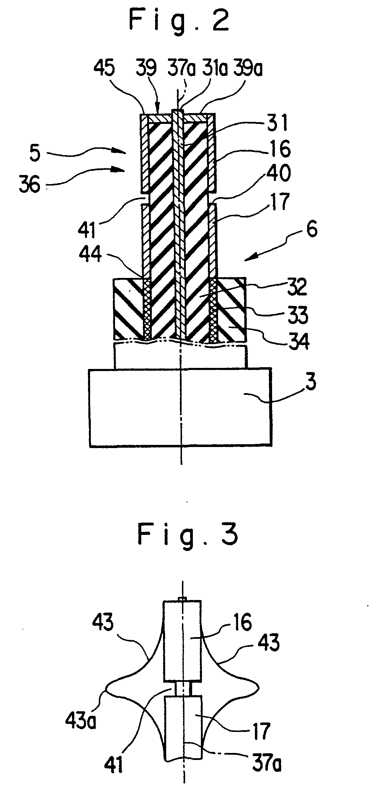

The antenna 5 having the electrodes of identical shapes and the coaxial cable 6 will be explained in detail hereinafter referring to the embodiments shown in Figs. 2 to 5.

The balloon 8 is secured at its base portion to the end 12 of the water-drain tube 15 and secured about at the center of its top end 18 to the top end of the microwave antenna 5.

It is preferred that the polymeric film of the balloon 8 is made of highly flexible material so that the film can be in close contact with the surface of the endotract at the lesion and that the film is made as thin as possible so that it may absorb less energy of the microwaves emitted or transmitted from the antenna 5, provided that the film has an elasticity sufficient to contain water therein under a certain pressure. In the case of using a thin rubber film for the balloon 8, the film thickness is, for instance, less than 0.5 mm (energy loss of about 30%) and, preferably, less than 0.1 mm (energy loss of about 10—15%). Although the balloon 8 in the illustrated embodiment is shaped such that it is expandable through an oval or elliptic shape to a generally spheric shape, it may take any other configuration. For instance, the balloon 8 may be protruded longitudinally at the top end 18 and, in this case, the top end of the antenna 5 may be inserted into but not fixed to the elongated recess of the top end 18. The film material for the balloon 8 is preferably rubbery polymer material, for example, natural rubber of synthetic rubber such as silicone rubber.

In order to transmit the microwaves emitted from the antenna 5 as effectively as possible to the lesion, the cooling medium flowing the inside of the balloon 8, preferably, comprises liquid medium at least mainly composed of water so that the emitted microwaves are transmitted therein substantially at the same wavelength as in the lesion. Purified water with less transmission loss such as absorption is more preferred.

The purified water as the cooling liquid flowing inside of the balloon 8 is kept at an appropriate temperature of about 0—45°C and, preferably, about 15—42°C so that the temperature at the lesion can be maintained at 42—45°C by the purified water in co-operation with the antenna 5.

Further, the flow rate of the cooling water is controlled by the throttling device 14. The throttling device 14 comprises a manually- or automatically-controlled valve, the opening degree of which can be adjusted continuously. The throttling device or valve 14 also serves to produce an adequate pressure within the balloon 8 so as to expand the balloon 8 into an intimate contact with the wall of the tract or lumen organ.

In the illustrated embodiment, the drain tube 15 is constituted as a device main body which is to be inserted through the tract or lumen and through which the coaxial cable 6 and the feed tube 11 are extended. Alternatively, the feed tube 11 may be modified to be constituted as the device main body while extending the drain tube 15 and the coaxial cable 6 the inside of the feed tube 11. In this modified embodiment, the base portion of the balloon 8 is secured to the end of the water-feed tube 11. Furthermore, the feed tube 11, the drain tube 15 and the coaxial cable 6 may be bundled in close contact together at their respective outer circumferential surfaces so that the three members form an elongated antenna device main body as a whole, with the balloon 8 being capped over the open ends of the tubes 11, 15 so as to surround the antenna 5, in the case where the assembly can be formed so smooth and thin as can be intaken, for example, from the mouth into the stomach.

The purified water may be used recyclically by connecting the drain tube 15 to the pump 10 by way of a reservoir not shown. Reference numeral 20 in the draining denotes sealed portions to prevent the leak of the water.

A thermosensor or temperature detector 21 is fixed to the outer surface of the central portion of the circumferential wall of the balloon 8 to detect the temperature at the film or membrane surface of the balloon 8, that is, the temperature at the inner surface of the wall of the tract organ. The thermosensor 21 may be a thermocouple or thermistor. The lead 22 for the thermosensor 21 is disposed along the outer surface of the drain tube 15 constituting the device main body in the illustrated embodiment, it may, however, be passed through the inside of the drain tube 15 as the device main body if desired. The average output from the oscillator 3 and the temperature of the cooling water fed to the balloon 8 are controlled depending on the output signal from the thermosensor 21, that is, on the temperature detected by the sensor 21 so that the temperature of the lesion may be kept at about 42—45°C. The temperature control may be carried out automatically by a suitable control means.

In the endotract antenna device for hyperthermia 1 constituted as described above, since the microwave radiation antenna 5 is disposed to the inside of the balloon 8 through which the cooling liquid passes, the balloon 8 can be deformed just corresponding to the uneven inner profile of the wall of the tract organ and put to an intimate fitting with the inner surface of the wall, that is, the surface of the lesion by the control of the flow rate and/or the pressure of the cooling liquid flowing inside of the balloon 8, whereby the microwave emitted from the antenna 5 can be transmitted with little transmission loss to the lesion through the purified water in the chamber 7 and the thin film of the balloon 8. Furthermore, control for the temperature and the flow rate of the cooling water flowing inside of the balloon 8 can ensure the hyperthermia therapy while preventing localized over-heating and maintaining the temperature of the wide lesion area at a temperature of between 42—45°C.

As described above, according to this invention, since the endotract antenna device for hyperthermia comprises a microwave radiation antenna, a balloon-like member made of a polymer thin film and surrounding the antenna, and means for feeding and draining a cooling liquid to and from the inside of the balloon-member, the energy of the microwaves emitted from the antenna can be effectively and uniformly given to the lesion in the tract or lumen organ, thereby enabling to warm the lesion effectively.

Fig. 2 shows an embodiment of the microwave antenna contained in the endotract antenna device for hyperthermia. In the Figure 2, the coaxial cable 6 for the microwave transmission comprises a central or inner conductor 31, an insulator 32, an outer conductor 33 and a protection cover 34. The cable 6 is connected at one end thereof to the microwave oscillator or generator 3 capable of continuously generating microwaves at a frequency, for example, of 915 MHz.

The coaxial cable 6 is preferably designed, for enabling effective transmission of the microwaves and easy insertion into the tract where the lesion is located, such that the cable has an outer diameter of about 2—10 mm and comprises the central conductor 31 in the form a single wire or twisted wires made of silver-plated copper wire, the insulator 32 made of a polymer material with less dielectric loss and the outer shielding conductor 33 in the form of a braided tube or helically wound braided cable made of silver-plated annealed copper wires. Further, the protection cover 34 for the cable is, preferably, made of such a polymer material as exhibiting no toxicity when put to be in contact with the tract, for example, fluoro resin, polyvinyl chloride and polyethylene.

The antenna 5 for microwave transmission or radiation is formed at the top end side 36 of the coaxial cable 6 by removing the protective cover 34 at its top end side 36. The antenna 5 comprises the tubular conductor 17 of about $\lambda/4$ in length ($\lambda$ is a wave length of the microwave in the chamber 7 in Fig. 1) integrally formed with the outer conductor 33, and another tubular conductor 16, of the same shape as the tubular conductor 17, which is electrically connected at its top end 39 by means of a soldered portion 39a to the central conductor 31 and attached to the insulator 32 with a slight axial gap 41 from an extended end 40 of the tubular conductor 17. The length for each of the tubular conductors 17 and 16 may be an integral multiple more than one, that is, 2, 3, 4, ... times of the length $\lambda/4$.

The antenna 5 may be fabricated, for instance, by the following procedures. At first the central conductor 31 is exposed to about 1 mm at the top end 39 of the coaxial cable 6 (having an outer diameter, e.g., of 3 mm) and the protective cover 34 is removed by the length of about $\lambda/2$ (e.g., about 22 mm for the case of 915 MHz) at the top end 36 to reveal or expose the outer conductor 33 composed of the braided tubes. Then, the exposed portion of the conductor 33 about $\lambda/2$ in length is entirely impregnated with a solder to form a tubular body uniformly deposited or coated with the solder over the exposed portion. Thereafter the tubular body is divided into two exposed tubular conductors 16 and 17 each of a same length by forming a circular slit at a position of about $\lambda/4$ (for example, of about 11 mm) from the top end of the tubular body. Then, the tubular conductor 16 at the top end side 39 is drawn by about 1 mm toward the top end 39 and electrically connected by means of soldering with the projected end 31a of the central conductor 31.

The central conductor 31 and the tubular conductor 19 on the side of the top end 39 may be electrically connected not only by way of soldering but also with an annular conductor plate made of copper or the like. The tubular conductor 16 may be formed by fitting a tubular conductor of about $\lambda/4$ in length made of copper or like other metal over the insulator 32, instead of using the braided tubes impregnated with the solder. Furthermore, the tubular conductor may have an end wall formed with an aperture through which central conductor 31 is fitted. In addition, the other tubular conductor 17 may also be formed with a copper tube or the like, which is fitted over the insulator 32, electrically connected with the outer conductor 33 and secured to the coaxial cable 6.

At first, the braided portion for forming the tubular conductor 17 may be exposed to more than $\lambda/4$ in length and impregnated with solder or the like to form an exposed tubular conductor of more than $\lambda/4$ in length. In this case, insulating resin adhesives or the like can be applied for covering a portion of the tubular conductor near the opening end of the protective cover 34 in such a way that the tubular conductor may be exposed over length of about $\lambda/4$ and that liquid materials may not intrude between the tubular conductor 17 and the outer cover 34 to the inside of the axial cable 6.

When the antenna 5 was connected to the microwave oscillator 3 operating at 915 MHz while matching an impedance as long as possible, the antenna had characteristics of an output power of 15 watt and a reflection factor of 5%. The microwave radiation pattern at 915 MHz from the antenna 5 immersed in a physiological saline water was as shown in Fig. 3. Fig. 3 shows an isothermal curve at 40°C by the lines 43, which are rotationally symmetrical about the center line 37a of the antenna 5 and are also symmetrical for the mirror operation or reflection in an imaginary plane passing through the gap 41 in perpendicular to the line 37a. The line 43 exhibits peaks 43a at portions facing to the gap 41. Accordingly, effective therapy can be carried out by disposing the antenna 5 so as to oppose the peak 43a, that is, the gap 41 to the lesion, because the microwave antenna 5 is formed vertically symmetrical with respect to the gap 41 and because the electric power is fed to the tubular conductors 16 and 17 of the antenna 5 from the separated side ends 44 and 45 respectively.

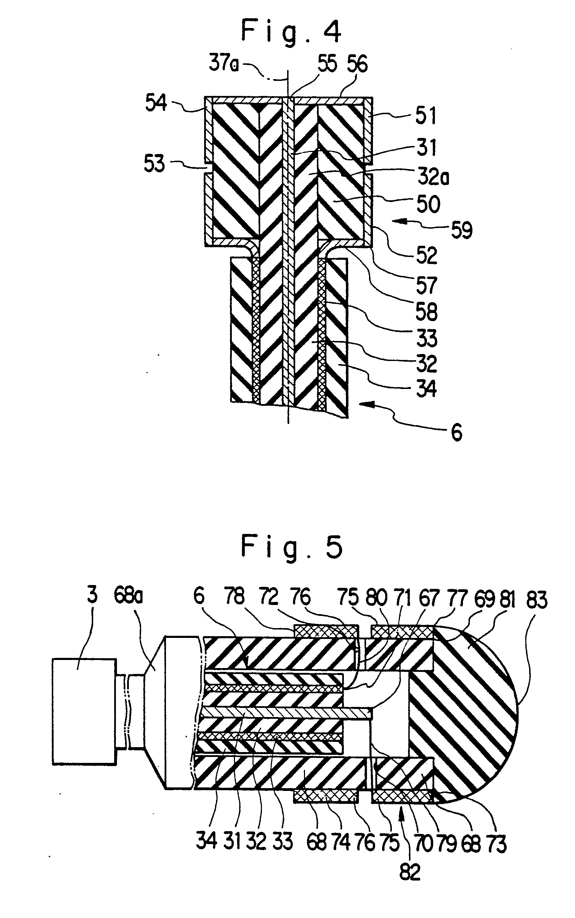

Instead of forming the microwave antenna directly on the extension of the intermediate insulator 32 for the coaxial cable 6 as shown in Fig. 2, a microwave antenna 59 may be modified as shown in Fig. 4, in which a cylindrical insulating resin tube 50 of about $\lambda/2$ in length is fitted over the extension 32a of the insulator 32 slightly longer than $\lambda/2$ in length and cylindrical metal tubular conductors 51, 52 made of copper and the like of about $\lambda/4$ in length, respectively, are fitted while separated from each other by a gap 53 over the resin tube 50. A central conductor 31 of a coaxial cable 6 is electrically connected by means of soldering or the like to the top end 54 of the tubular conductor 51 remote from the tubular conductor 52 by way of a disc 56 made of copper or like other metal and formed with an aperture 55. The outer conductor 33 is electrically connected by means of soldering or the like to the end 57 of the tubular conductor 52 remote from the tubular conductor 51 by way of conductor wires 58. The conductor wires 58 may be formed by partially releasing and spreading the braided tube of the outer conductor 33. The characteristics and the radiation pattern of the antenna 59 were the same as those of the antenna 5.

As described above, since the microwave antenna contained within the balloon of the endotract antenna device for hyperthermia according to this invention comprises two identically-shape elongated conductors of about $\lambda/4$ in length formed at the top end of a coaxial cable on a straight line and slightly spaced apart from each other, the radiation energy is made at the maximum near the separated portion or gap between the two conductors and the effective therapy can be carried out by opposing the separated portion to the lesion.

Fig. 5 shows another modified embodiment of the microwave antenna according to this invention, in which a flexible coaxial cable 6 for transmitting microwaves has the same constitution as the coaxial cable shown in Fig. 2 and it is connected at one end thereof to a microwave oscillator 3 capable of continuously generating microwaves. The central conductor 31 of the coaxial cable is slightly projected at the top end 67 thereof.

A flexible tube 68 made of flexible polymer material such as soft polyvinyl chloride is supported at its base end 68a to the top end of the coaxial cable 6 and the top end of the flexible tube 68 is projected by about $\lambda/4$ or more in length from the coaxial cable 6. The tube 68 has a radial penetrating hole 70 at the position of about $\lambda/4$ from the top end 69 and another radial penetrating hole 72 at the position of about $\lambda/4$ or more from the top end, on the opposite side to the hole 70 and generally opposed to the top end 71 of the outer conductor 33 of the coaxial cable 6.

While the flexible tube 68 has a larger inner diameter than the outer diameter for the coaxial cable 6 so as to be kept highly flexible the inner diameter of the tube 68 may be the same as the outer diameter of the coaxial cable 6, provided that the coaxial cable 6 can be inserted and that the assembly of the tube 68 and the cable is kept highly flexible.

The flexible tube 68 may be made of flexible polymer material such as rubber and high pressure process polyethylene.

Electrically conductive and flexible cylindrical members 73 and 74 are fitted over the flexible tube 68 while spaced apart, for example, by about 1 mm from each other so that their opposing ends 75 and 76 are situated at the openings of the holes 70 and 72 respectively. The hollow cylindrical members 73 and 74 are made of braided tubes made of, for example, copper wires braided in a hollow cylindrical configuration and have about $\lambda/4$ in length (for example, 11 mm at 915 MHz) in the condition fitted to the tube 68. The cylindrical members 73 and 74 are secured at both ends 75, 77 and 76, 78 respectively to the tube 68. The cylindrical member 73 is electrically connected at its one end 75 to the top end 67 of the central conductor 31 by way of a conductor wire or lead 79 passed through the hole 70, while the cylindrical member 74 is electrically connected at its one end 76 to the top end 71 of the outer conductor 33 by way of a conductor wire or lead 80 passed through the hole 72.

A cap 81 rounded at the top end is mounted to the top end 69 of the flexible tube so as to facilitate the insertion of the flexible microwave antenna 82, into the tract, comprising the flexible and electrically conductive member 73 of about $\lambda/4$ in length connected to the central conductor 31 and the flexible and electrically conductive cylindrical member 74 of about $\lambda/4$ in length connected to the outer conductor 33. The cap 81 preferably made of flexible polymer material prevents the body fluid from intruding to the inside of the tube 68 in cooperation with the resin adhesives filled in the holes 70 and 72.

The flexible microwave antenna 82 having the foregoing constitution may be assembled, for example, by fitting the braided tubes 74 and 73 over the tube 68 in which the holes 70 and 72 are formed at predetermined positions, securing the tube 68 over the top end of the coaxial cable 6, connecting the braided tubes 74 and 73 with the outer and the central conductors 33 and 31 by way of the conductor wires 80 and 79 by means of soldering or the like, securing the braided tubes 74 and 73 to the tube 68 so as to have the predetermined length of $\lambda/4$ at the position spaced apart by about 1 mm from each other, further securing the conductor wires 80 and 79 to the holes 72 and 70 respectively and mounting the cap 81 over the tube 68 and the braided tube 73.

Alternatively, the outer conductor 33 may be connected electrically to the flexible hollow cylindrical member 73 at the top end, while the central conductor 31 may be connected electrically to the other cylindrical member 74. The position and the number for the penetrating holes 70 and 72 in the tube 68 are determined in view of the current distribution and not restricted to the illustrated positions generally diametrically opposing each other.

In a case where it is desired to constitute a particularly thin antenna or the like, a pair of fine or thin flexible braided tubes 73 and 74 may be directly fitted around the outer circumference of the flexible protective cover 34 of the coaxial cable 6. Furthermore, the tubular braided conductor of the outer conductor 33 may be used directly as a pair of flexible braided tubes 73 and 74.

The flexible microwave antenna 82 having the foregoing constitution can be in close contact with the tract wall at the lesion and deeply inserted into the tract because of its flexibility, and thus can be effectively applied to the therapy of the lesion. In addition, in a case where the antenna 82 of 18 W in output power was deformed or curved by transversely or laterally displacing the top end 83 of the antenna 82 by 10 mm from its straightly extended position, the reflection factor can be maintained not more than 5—7%. This result shows that, although the antenna deformation has been avoided in the prior art fearing the degradation in the antenna characteristic, the flexible antenna 82 according to this invention can be effectively applied to the hyperthermia therapy with less fear of degrading the characteristic.

As described above, according to this invention, since the flexible microwave antenna contained in the endotract heating antenna device comprises flexible cylindrical members made of electrically conductive material mounted to the outer circumference of a rod-like member made of flexible and insulating material, it can be effectively applied for the therapy of the lesion in the tract or lumen.

The microwave antenna described herein above can be used not only in a condition contained in the endotract antenna device for hyperthermia but also in a condition in direct contact with the body fluid or the lesion.

**Claims**

1. An endotract antenna device for hyperthermia comprising an antenna (5) for radiating microwaves, and an envelope (8) made of a polymer material surrounding said antenna (5), characterised in that the envelope (8) is a balloon-like member made of a flexible and elastic polymeric thin film and forming a chamber (7) of variable volume, and by means (11, 15) for feeding and draining a cooling liquid to and from the inside of said balloon-like member (8).

2. An antenna device according to claim 1, characterised in that the microwave radiating antenna comprises two elongated and identically-shaped conductors (17, 16; 51, 52) arranged on a straight line and slightly spaced apart from each other, each having a length equal to an integral multiple of about 1/4 of a wavelength of the microwaves to be emitted from said antenna.

3. An antenna device according to claim 2, characterised in that the two identically-shaped conductors (17, 16; 51, 52) are formed at the top end of a coaxial cable (6), and comprise a tubular conductor of a length of about 1/4 of the wavelength electrically connected to one of a central conductor (31) and an outer conductor (33) of the coaxial cable, and another tubular conductor of a length of about 1/4 of the wavelength electrically connected to the other one of the outer conductor (33) and the central conductor (31) of the coaxial cable (6).

4. An antenna device according to claim 3, characterised in that said two tubular conductors (17, 16) are attached to an insulator (32) between the central conductor (31) and the outer conductor (33).

5. An antenna device according to claim 3, characterised in that the microwave radiation antenna (5) includes a hollow cylindrical insulating resin tube (50) having a length of about 1/2 of the wavelength fitted over an insulator (32) disposed between the central conductor (31) and the outer conductor (33) of the coaxial cable (6), and in that said two tubular conductors (51, 52) forming a gap therebetween fit around the resin tube (50), each of said two tubular conductors (51, 52) being made of a cylindrical metallic tube.

6. An antenna device according to any one of claims 3 to 5, characterised in that the coaxial cable (6) comprises the central conductor (31) which is composed of a single wire or twisted wires made of a silver-plated copper wire, the insulator (32) between the central conductor (31) and the outer conductor (33) and which is made of a polymer material with less dielectric loss, the outer shielding conductor (33) which is composed of a braided tube or a helically wound braided cable made of silver-plated annealed copper wires, and a protective cover (34) which is made of a polymer material and is disposed to the outer side of the outer conductor.

7. An antenna device according to claim 1, characterised in that the microwave radiation antenna comprises a pair of flexible tubular members (73, 74) made of electrically conductive material for microwave radiation mounted on the outer circumference of a rod-like member (68) made of a flexible and insulating material.

8. An antenna device according to claim 7, characterised in that the rod-like member (68) is hollow and contains an end portion of a coaxial cable (6) including a central conductor (31), an outer conductor (33), an insulator (32) between the central conductor and the outer conductor, and a protective cover (34) outside the outer conductor, the coaxial cable (6) being electrically connected to the tubular members (75, 76) and the rod-like member (68) projecting beyond the end portion of the coaxial cable (6).

9. An antenna device according to claim 7 or 8, characterised in that the flexible tubular members (73, 74) are each braided metallic tubes of a length of about 1/4 of the wavelength of the microwaves to be emitted from the antenna and are fitted over the rod-like member (68) slightly spaced apart from each other.

10. An antenna device according to any preceding claim, characterised in that said radiation antenna (5) is connected by way of a coaxial cable (6) to a microwave oscillator (3).

11. An antenna device according to any preceding claim, characterised in that the free end of the radiation antenna (5) is attached to a central portion (18) of a top end of said balloon-like member (8).

12. An antenna device according to any preceding claim, characterised in that the polymeric film is made of rubber.

13. An antenna device according to claim 12, characterised in that the rubber is synthetic rubber.

14. An antenna device according to claim 12, characterised in that the rubber is natural rubber.

15. An antenna device according to any preceding claim, characterised in that said feeding and draining means comprises a feeding tube (11) communicated at one end thereof with said balloon-like member (8) so as to feed cooling liquid to the inside of said balloon-like member and a draining tube (15) communicated at one end thereof with said balloon-like member (8) so as to drain the cooling liquid from said balloon-like member.

16. An antenna device according to claim 15, characterised in that said feeding and draining means further includes a pump (10) connected to another end of the feeding tube (11) so as to supply the cooling liquid by way of the feeding tube (11) to the inside of said balloon-like member (8).

17. An antenna device according to claim 15 or 16 characterised in that said feeding and draining means further comprises a throttling means (14) for controlling the flow rate or pressure of the cooling liquid flowing to the inside of said balloon-like member (8).

18. An antenna device according to claim 17 characterised in that the throttling means (14) is disposed at another end of the draining tube (15).

19. An antenna device according to claim 15, 16, 17 or 18 characterised in that the balloon-like member (8) is liquid-tightly attached to the one end of the draining tube (15) the feeding tube (11) is inserted into the draining tube (15) through a side thereof at the other end, and a transmission line (6) for connecting a microwave oscillator (3) with said radiation antenna (5) is inserted in the draining tube (15).

20. An antenna device according to claim 15, 16, 17 or 18 characterised in that the balloon-like member (8) is liquid-tightly attached to the one end of the feeding tube (11) the draining tube (15) is inserted in the feeding tube (11) through a side thereof at the other end, and a transmission line (6) for connecting a microwave oscillator (3) with said radiation antenna (5) is inserted in the feeding tube (11).

21. An antenna device according to any of claims 15 to 20 characterised in that said feeding and draining means comprises means for controlling the temperature of the cooling liquid to be supplied by way of the feeding tube (11) to said balloon-like member (7).

22. An antenna device according to any preceding claim characterised in that the cooling liquid is water.

23. An antenna device according to claim 22 characterised in that the water is purified water.

24. An antenna device according to any preceding claim, characterised by a thermosensor (21) being disposed on the surface of the balloon-like member.

**Patentansprüche**

1. Antennenvorrichtung zur inneren Hyperthermiebehandlung von Körperorganen, die eine Antenne (5) zur Abstrahlung von Mikrowellen und eine diese Antenne (5) umgebende Umhüllung (8) aus einem Polymermaterial enthält, dadurch gekennzeichnet, daß die Umhüllung (8) ein ballonartiges Element aus einem flexiblen und elastischen, dünnen Polymerfilm ist und eine Kammer (7) von veränderlichen Volumen bildet, und daß die Vorrichtung Mittel (11, 15) für die Zuführung und Ableitung einer Kühlflüssigkeit zu und aus dem Inneren dieses ballonartigen Elements (8) aufweist.

2. Antennenvorrichtung nach Anspruch 1, dadurch gekennzeichnet, daß die Mikrowellen abstrahlende Antenne zwei längliche und identisch gestaltete Leiter (17, 16; 51, 52) enthält, die auf einer geraden Linie angeordnet und geringfügig voneinander beabstandet sind, wobei die Länge eines jeden Leiters ein ganzzahliges Vielfaches von etwa 1/4 der Wellenlänge der von dieser Antenne auszusendenden Mikrowellen beträgt.

3. Antennenvorrichtung nach Anspruch 2, dadurch gekennzeichnet, daß die zwei identisch geformten Leiter (17, 16; 51, 52) an dem Ende eines Koaxialkabels (6) ausgebildet sind und daß sie bestehen aus einem Hohlleiter mit einer Länge von etwa 1/4 der Wellenlänge, welcher elektrisch leitend verbunden ist mit dem zentralen Leiter (31) oder mit dem Außenleiter (33) des Koaxialkabels, sowie aus einem weiteren Hohlleiter mit einer Länge von etwa 1/4 der Wellenlänge, welcher elektrisch leitend verbunden ist mit dem Außenleiter (33) oder dem zentralen Leiter (31) des Koaxialkabels (6).

4. Antennenvorrichtung nach Anspruch 3, dadurch gekennzeichnet, daß diese beiden Hohlleiter (17, 16) an einem zwischen dem zentralen Leiter (31) und dem Außenleiter (33) liegenden Isolator (32) befestigt sind.

5. Antennenvorrichtung nach Anspruch 3, dadurch gekennzeichnet, daß die Mikrowellen-Antenne (5) eine hohle zylindrische, isolierende Röhre (50) aus Kunstharz einschließt, die eine Länge von etwa 1/2 der Wellenlänge besitzt und die über einen, zwischen dem zentralen Leiter (31) und dem Außenleiter (33) des Koaxialkabels (6) angeordneten Isolator (32) geschoben ist, und daß diese beiden Hohlleiter (51, 52), die zwischen sich einen Spalt bilden, um die Harzröhre (50) passend angeordnet sind, wobei jeder dieser beiden Hohlleiter (51, 52) aus einem zylindrischen Metallrohr hergestellt ist.

6. Antennenvorrichtung nach einem der Ansprüche 3 bis 5, dadurch gekennzeichnet, daß das Koaxialkabel (6) besteht aus:

dem zentralen Leiter (31), der aus einem einzelnen Draht oder aus verdrillten Drähten aus versilbertem Kupferdraht besteht,

dem Isolator (32), der zwischen dem zentralen Leiter (31) und dem Außenleiter (33) angeordnet ist und aus einem Polymermaterial mit geringeren dielektrischen Verlusten hergestellt ist,

dem äußeren, abschirmenden Leiter (33), der aus einem Metallschlauch oder einem spiralig umwickelten Kabel aus versilberten, geglühten Kupferdrähten besteht,

sowie aus einem Schutzüberzug (34), der aus einem polymeren Material hergestellt und auf die Außenseite des Außenleiters aufgebracht ist.

7. Antennenvorrichtung nach Anspruch 1, dadurch gekennzeichnet, daß die Mikrowellen-Antenne ein Paar flexibler rohrförmiger Elemente (73, 74) aus elektrisch leitendem Material zur Mikrowellenabstrahlung enthält, die auf dem äußeren Umfang eines aus flexiblem und isolierendem Material hergestellten, stabförmigen Elements (68) angeordnet sind.

8. Antennenvorrichtung nach Anspruch 7, dadurch gekennzeichnet, daß das stabförmige Element (68) hohl ist und einen Endabschnitt eines Koaxialkabels (6) enthält, zu dem ein zentraler Leiter (31), ein äußerer Leiter (33), ein Isolator (32) zwischen dem zentralen Leiter und dem äußeren Leiter und ein Schutzüberzug (34) auf der Außenseite des äußeren Leiters gehören, wobei das Koaxialkabel (6) elektrisch leitend mit den rohrförmigen Elementen (75, 76) verbunden ist und das stabförmige Element (68) über den Endabschnitt des Koaxialkabels (6) hinausragt.

9. Antennenvorrichtung nach Anspruch 7 oder 8, dadurch gekennzeichnet, daß die flexiblen rohrförmigen Elemente (73, 74) beide Metallschläuche mit einer Länge von etwa 1/4 der Wellenlänge der von der Antenne auszusendenden Mikrowellen sind und daß sie in geringem Abstand voneinander passend über dem stabförmigen Element (68) angeordnet sind.

10. Antennenvorrichtung nach einem der vorstehenden Ansprüche, dadurch gekennzeichnet, daß diese Abstrahlungs-antenne (5) über ein Koaxialkabel (6) an einen Mikrowellen-Oszillator (3) angeschlossen ist.

11. Antennenvorrichtung nach einem der vorangegangenen Ansprüche, dadurch gekennzeichnet, daß das freie Ende der Abstrahlungsantenne (5) an einem zentralen Abschnitt (18) eines Scheitels dieses ballonartigen Elements (8) befestigt ist.

12. Antennenvorrichtung nach einem der vorangegangenen Ansprüche, dadurch gekennzeichnet, daß der Polymerfilm aus Gummi besteht.

13. Antennenvorrichtung nach Anspruch 12, dadurch gekennzeichnet, daß der Gummi synthetischer Gummi ist.

14. Antennenvorrichtung nach Anspruch 12, dadurch gekennzeichnet, daß der Gummi Naturgummi ist.

15. Antennenvorrichtung nach einem der vorangegangenen Ansprüche, dadurch gekennzeichnet, daß diese Mittel zur Zuleitung und Ableitung aus einer Zuleitung (11), deren eines Ende mit diesem ballonartigen Element (8) verbunden ist, um Kühlflüssigkeit dem Inneren dieses ballonartigen Elements zuzuführen, und aus einer Ableitung (15) bestehen, deren eines Ende mit diesem ballonartigen Element (8) verbunden ist, um die Kühlflüssigkeit aus diesem ballonartigen Element abzuziehen.

16. Antennenvorrichtung nach Anspruch 15, dadurch gekennzeichnet, daß zu diesen Mitteln zur Zuleitung und Ableitung ferner eine Pumpe (10) gehört, die an das andere Ende der Zuleitung (11) angeschlossen ist, um die Kühlflüssigkeit über die Zuleitung (11) ins Innere dieses ballonartigen Elements (8) zu fördern.

17. Antennenvorrichtung nach Anspruch 15 oder 16, dadurch gekennzeichnet, daß diese Mittel zur Zuleitung und Ableitung außerdem eine Drossel (14) zur Regulierung der Fließgeschwindigkeit oder des Drucks der durch das Innere dieses ballonartigen Elements (8) fließenden Kühlflüssigkeit enthält.

18. Antennenvorrichtung nach Anspruch 17, dadurch gekennzeichnet, daß die Drossel (14) an einem anderen Ende der Ableitung (15) angebracht ist.

19. Antennenvorrichtung nach Anspruch 15, 16, 17 oder 18, dadurch gekennzeichnet, daß das ballonartige Element (8) flüssigkeitsdicht an dem einen Ende der Ableitung (15) befestigt ist, die Zuleitung (11) in die Ableitung (15) an deren anderem Ende seitlich eingeführt ist und eine Übertragungsleitung (6) zur Verbindung eines Mikrowellen-Oszillators (3) mit dieser Abstrahlungsantenne (5) in die Ableitung (15) eingeführt ist.

20. Antennenvorrichtung nach Anspruch 15, 16, 17 oder 18, dadurch gekennzeichnet, daß das ballonartige Element (8) flüssigkeitsdicht an dem einen Ende der Zuleitung (11) befestigt ist, die Ableitung (15) in die Zuleitung (11) an deren anderem Ende seitlich eingeführt ist und eine Übertragungsleitung (6) zur Verbindung eines Mikrowellen-Oszillators (3) mit dieser Abstrahlungsantenne (5) in die Zuleitung (11) eingeführt ist.

21. Antennenvorrichtung nach einem der Ansprüche 15 bis 20, dadurch gekennzeichnet, daß diese Mittel zur Zuleitung und Ableitung Mittel zur Regulierung der Temperatur der über die Zuleitung (11) zu diesem ballonartigen Element (8) zu fördernden Kühlflüssigkeit aufweisen.

22. Antennenvorrichtung nach einem der vorangegangenen Ansprüche, dadurch gekennzeichnet, daß die Kühlflüssigkeit Wasser ist.

23. Antennenvorrichtung nach Anspruch 22, dadurch gekennzeichnet, daß das Wasser gereinigt ist.

24. Antennenvorrichtung nach einem der vorangegangenen Ansprüche, dadurch gekennzeichnet, daß ein Thermofühler (21) auf der Oberfläche des ballonartigen Elements angeordnet ist.

## Revendications

1. Un dispositif d'antenne endotract pour hyperthermie, comprenant une antenne (5) pour la radiation de micro-ondes et une enveloppe (8) en matériau polymère entourant la dite antenne (5), le dispositif étant caractérisé en ce que l'enveloppe (8) est un élément de type ballon réalisé à partir d'un mince film polymère flexible et élastique formant une chambre (7) de volume variable, des moyens (11, 15) étant prévus pour alimenter un liquide de refroidissement à l'intérieur du dit élément de type ballon (8) et pour évacuer ce liquide de l'intérieur de cet élément.

2. Un dispositif d'antenne selon la revendication 1, caractérisé en ce que l'antenne pour la radiation de micro-ondes comprend deux conducteurs allongés et profilés de manière identique (17, 16; 51, 52) disposés en ligne droite et légèrement distants l'un de l'autre, chacun de ces conducteurs ayant une longueur égale à un multiple intégral d'environ 1/4 d'une longueur d'onde des micro-ondes devant être émises à partir de la dite antenne.

3. Un dispositif d'antenne selon la revendication 2, caractérisé en ce que les deux conducteurs profilés de manière identique (17, 16; 51, 52) sont formés à l'extrémité supérieure d'un câble coaxial, (6), et comprennent un conducteur tubulaire d'une longueur d'environ 1/4 de la longueur d'onde, raccordé électriquement à un des conducteurs centraux (31) et à un des conducteurs extérieurs (33) du câble coaxial, et un autre conducteur tubulaire d'une longueur d'environ 1/4 de la longueur d'onde, raccordé électriquement à l'autre des conducteurs éxtérieurs (33) et à l'autre des conducteurs centraux (31) du câble coaxial (6).

4. Un dispositif d'antenne selon la revendication 3, caractérisé en ce que les deux dits conducteurs tubulaires (17, 16) sont fixés à un isolateur (32) entre le conducteur central (31) et le conducteur extérieur (33).

5. Un dispositif d'antenne selon la revendication 3, caractérisé en ce que l'antenne de radiation de micro-ondes (5) inclue un tube résine isolant cylindrique creux ayant une longueur d'environ 1/2 de la longueur d'onde, monté sur un isolateur (32) disposé entre le conducteur central (31) et le conducteur extérieur (33) du câble coaxial (6), les deux dits conducteurs tubulaires (51, 52) formant dans cet espace entre le conducteur central (31) et le conducteur extérieur (33) un entrefer prévu pour entourer le tube résine (50), chacun des deux dits conducteurs tubulaires (51, 52) consistant en un tube métallique cylindrique.

6. Un dispositif d'antenne selon une quelconque des revendications 3 à 5, caractérisé en ce que le câble coaxial (6) comprend le conducteur central (31) qui est composé d'un fil unique ou de fils retordus consistant en fil cuivre plaqué argent, l'isolateur (32) entre le conducteur central (31) et le conducteur extérieur (33) étant réalisé à partir de matériau polymère avec perte diélectrique réduite, le conducteur de blindage extérieur (33) étant composé d'un tube tressé ou d'un câble tressé enroulé hélicoïdalement consistant en fils de cuivre recuit trempé plaqué argent, et une gaîne protectrice (34) étant réalisée à partir de matériau polymère et disposée sur le côté extérieur du conducteur extérieur.

7. Un dispositif d'antenne selon la revendication 1, caractérisé en ce que l'antenne de radiation de micro-ondes comprend une paire d'éléments tubulaires flexibles (73, 74) en matériau électriquement conducteur pour la radiation des micro-ondes, ces éléments étant montés sur la circonférence extérieure d'un élément en forme de tige (68) réalisé à partir d'un matériau souple et isolant.

8. Un dispositif d'antenne selon la revendication 7, l'élément en forme de tige (68) est creux et contient une partie d'extrémité d'un câble coaxial (6) incluant: un conducteur central (31); un conducteur extérieur (33); un isolateur (32) entre le conducteur central et le conducteur extérieur; et une gaîne protectrice (34) à l'extérieur du conducteur extérieur, le câble coaxial (6) étant raccordé électriquement aux éléments tubulaires (75, 76) et l'élément en forme de tige (68) dépassant au-delà de la partie d'extrémité du câble coaxial (6).

9. Un dispositif d'antenne selon la revendication 7 ou 8, caractérisé en ce que les éléments tubulaires flexibles (73, 74) sont chacun des tubes métalliques tressés d'une longueur d'environ 1/4 de la longueur d'onde des ondes devant être émises à partir de l'antenne et sont montés légèrement distants l'un de l'autre sur l'élément en forme de tige (68).

10. Un dispositif d'antenne selon une quelconque des revendications précédentes, caractérisé en ce que la dite antenne à radiations (5) est raccordée au moyen d'un câble coaxial (6) à un oscillateur à micro-ondes (3).

11. Un dispositif d'antenne selon une quelconque des revendications précédentes, caractérisé en ce que l'extrémité libre de l'antenne à radiations (5) est fixée à une partie centrale (18) d'une extrémité supérieure du dit élément en forme de ballon.(8).

12. Un dispositif d'antenne selon une quelconque des revendications précédentes, caractérisé en ce que le film polymère est en caoutchouc.

13. Un dispositif d'antenne selon la revendication 12, caractérisé en ce que le caoutchouc est du caoutchouc synthétique.

14. Un dispositif d'antenne selon la revendication 12, caractérisé en ce que le caoutchouc est du caoutchouc naturel.

15. Un dispositif d'antenne selon une quelconque des revendications précédentes, caractérisé en ce que le dit moyen d'alimentation et d'évacuation comprend un tuyau d'alimentation (11) communiquant à une de ses extrémités avec le dit élément en forme de ballon (8) de manière à alimenter le liquide de refroidissement à l'intérieur du dit élément en forme de ballon, et un tuyau d'évacuation (15) communiquant à une de ses extrémités avec le dit élément en forme de ballon (8) de manière à évacuer le liquide de refroidissement à partir du dit élément en forme de ballon.

16. Un dispositif d'antenne selon la revendication 15, caractérisé en ce que le dit moyen d'alimentation et d'évacuation inclue en outre une pompe (10) reliée à une autre extrémité du tube d'alimentation (11) de manière à fournir, par l'intermédiaire du tube d'alimentation (11), le liquide de refroidissement à l'intérieur du dit élément en forme de ballon (8).

17. Un dispositif d'antenne selon la revendication 15 ou 16, caractérisé en ce que le dit moyen d'alimentation et d'évacuation comprend en outre un système d'étranglement (14) pour contrôler le régime de débit ou la pression du liquide de refroidissement s'écoulant à l'intérieur du dit élément en forme de ballon (8).

18. Un dispositif d'antenne selon la revendication 17, caractérisé en ce que le système d'étranglement (14) est disposé à une autre extrémité du tube d'evacuation (15).

19. Un dispositif d'antenne selon la revendication 15, 16, 17 ou 18, caractérisé en ce que l'élément en forme de ballon (8) est fixé étanche au liquide à une des extrémités du tube d'évacuation (15), le tube d'alimentation (11) étant inséré dans le tube d'évacuation (15) par un côté de celui-ci à l'autre extrémité, et une ligne de transmission (6) pour le raccordement d'un oscillateur micro-onde (3) à la dite antenne de radiation (5) étant insérée dans le tube d'évacuation (15).

20. Un dispositif d'antenne selon la revendication 15, 16, 17 ou 18, caractérisé en ce que l'élément en forme de ballon (8) est fixé étanche au liquide à une des extrémités du tube d'alimentation (11), le tube d'évacuation (15) étant inséré dans le tube d'alimentation (11) par un côté de celui-ci à l'autre extrémité, et une ligne de transmission (6) pour le raccordement d'un oscillateur micro-onde (3) à la dite antenne de radiation (5) étant insérée dans le tube d'alimentation (11).

21. Un dispositif d'antenne selon une quelconque des revendications 15 à 20, caractérisé en ce que le dit moyen d'alimentation et d'évacuation comprend un système pour contrôler la température du liquide de refroidissement devant être fourni par l'intermédiaire du tube d'alimentation (11) au dit élément en forme de ballon (7).

22. Un dispositif d'antenne selon une quelconque des revendications précédentes, caractérisé en ce que le liquide de refroidissement est de l'eau.

23. Un dispositif d'antenne selon la revendication 22, caractérisé en ce que l'eau est de l'eau épurée.

24. Un dispositif d'antenne selon une quelconque des revendications précédentes, caractérisé par un thermocapteur (21) disposé sur la surface de l'élément en forme de ballon.

Fig. 1

0 105 677

# Fig. 2

45 39 37a 31a 39a

5
36

41
44

31
16
40
17

6

32
33
34

3

# Fig. 3

43
16
43

43a

41
17

37a

# Fig. 4

# Fig. 5